# EUROPÄISCHE PATENTSCHRIFT

(11) **EP 1 371 384 B1**
(45) Veröffentlichungstag und Bekanntmachung des Hinweises auf die Patenterteilung: **14.06.2006**
(21) Anmeldenummer: 03007609.5
(22) Anmeldetag: 02.04.2003
(51) Int. Cl.: A61M 16/00

(54) **Vorrichtung zur Beatmung mit Steuerung**
A ventilator with control
Un respirateur controllable

(30) Priorität: 15.06.2002 DE 10226783
(43) Veröffentlichungstag der Anmeldung: 17.12.2003
(73) Patentinhaber: Weinmann Geräte für Medizin GmbH & Co. KG, 22525 Hamburg (DE)
(72) Erfinder: Wedler, Wolfgang, 21147 Hamburg (DE)
(74) Vertreter: Klickow, Hans-Henning

(56) Entgegenhaltungen:
- WO-A-97/16216
- WO-A-98/12965
- US-A- 5 953 713

## Beschreibung

Die Erfindung betrifft eine Vorrichtung zur Beatmung, die mit einer Steuereinheit sowohl zur Vorgabe eines assistierten Beatmungszyklusses unter Berücksichtigung mindestens eines von einem Sensor erfaßten Meßsignals als auch zur Vorgabe eines zeitgesteuerten mandatorischen Beatmungszyklusses versehen ist und bei der die Steuereinheit ein Steuerungsprogramm aufweist, das in einem Grundzustand eine Atemgasfördereinrichtung in Abhängigkeit von den vom Sensor erfaßten Meßsignalen ansteuert und bei der die Steuereinheit mindestens einen Komparator aufweist, der bei Erkennung eines Schlafzustandes ein Steuersignal bereitstellt, gemäß dem die Steuereinheit die Atemgasfördereinrichtung mindestens teilweise entsprechend einem an die Parameter der Spontanatmung angepaßten mandatorischen Beatmungszyklus ansteuert.

Komfortable moderne Beatmungsgeräte ermöglichen es häufig, sowohl eine assistierte, eine mandatorische als auch eine gemischt assistierte und mandatorische, kontrollierte Beatmung durchzuführen. Eine assistierte Beatmung, bei der der Beatmungszyklus des Beatmungsgerätes in Abhängigkeit von einer meßtechnisch erfaßten eigenen Atmungsaktivität des Patienten gesteuert wird, weist den Vorteil auf, daß der Patient die Durchführung der Beatmung als angenehm und nur wenig störend empfindet. Es erfolgt jedoch nur eine teilweise Entlastung des Patienten von der durchzuführenden Atemarbeit. Bei einer mandatorischen Beatmung erfolgt eine weitgehende Entlastung des Patienten von der Atemarbeit, der Patient empfindet eine derartige Atmung - speziell in der Anfangsphase einer Behandlung - jedoch als ausgesprochen störend und unangenehm. Häufig wird ebenfalls beobachtet, daß der Patient versucht, trotz einer mandatorischen Beatmung seinen eigenen Atmungsrhythmus gegen das Beatmungsgerät durchzusetzen. Eine derartige Betriebsweise führt zu einer zusätzlich erhöhten Atemarbeit des Patienten.

Zu einer Synchronisation des Betriebes des Beatmungsgerätes mit einem natürlichen Atmungsrhythmus des Patienten bei der assistierten Beatmung ist es bereits bekannt, meßtechnisch den Atemantrieb des Patienten zu erfassen und als Triggersignal für das Beatmungsgerät zu verwenden, um einen Druckaufbau auszulösen. Bei einer derartigen meßtechnischen Erfassung leistet in der Regel der Patient aber bereits ca. 30% seiner eigenen Atmungsarbeit, bis eine synchrone Gerätesteuerung erfolgen kann.

Die bekannten Vorrichtungen zur Beatmung sowie die Verfahren zur Ansteuerung derartiger Beatmungsgeräte erlauben es bislang noch nicht, in optimaler Weise die Vorteile einer assistierten Beatmung und einer mandatorischen Beatmung unter möglichst weitgehender Vermeidung der jeweiligen Nachteile miteinander zu kombinieren.

Aus der WO 97/16216 A ist eine Vorrichtung zur Beatmung bekannt, die mit einer Steuereinheit sowohl zur Vorgabe einer assistierten Beatmung als auch einer zeitgesteuerten mandatorischen Beatmung ausgestattet ist. Eine Aktivierung der jeweiligen Beatmungssteuerung erfolgt unter Berücksichtigung eines von einem Sensor erfaßten Meßsignals. Eine Auswertungseinheit ermittelt aus den Meßinformationen des Sensors einen Schlafzustand des Patienten und führt in Abhängigkeit vom Auswertungsergebnis die Beatmungssteuerung durch.

In der WO 98/12965 A wird die Verwendung einer Fuzzy-Logik zur Steuerung eines Beatmungsparameters beschrieben. Aus der US-A 5,953,713 ist die Verwendung von neuronalen Netzen zur Berechnung respiratorischer Parameter bekannt.

Aufgabe der vorliegenden Erfindung ist es, eine Vorrichtung der einleitend genannten Art derart zu konstruieren, daß ein funktionell optimierter Arbeitsablauf unterstützt wird.

Diese Aufgabe wird erfindungsgemäß dadurch gelöst, daß das Steuerprogramm der Steuereinheit derart ausgebildet ist, daß die Steuereinheit in einem Wachzustand des Patienten den assistierten Beatmungszyklus aktiviert, eine zyklische Erfassung und Auswertung des Meßsignals zur Erkennung eines Schlafzustandes des Patienten durchgeführt, bei Erkennung eines Schlafzustandes des Patienten automatisch den mindestes teilweise mandatorischen Beatmungszyklus aktiviert und bei Erkennung einer zunehmenden Schlaftiefe des Patienten den Anteil des mandatorischen Beatmungszyklus durch Erhöhung eines Atem-Minuten-Volumens vorgibt.

Die Durchführung des assistierten Beatmungszyklusses während eines Wachzustandes des Patienten führt zu einer vom Patienten bewußt wahrgenommenen angenehmen Betriebsweise des Beatmungsgerätes. Die zyklische Erfassung der Parameter zur Detektion eines Schlafzustandes und die entsprechende Auswertung haben zur Folge, daß frühzeitig und automatisch eine Überleitung der Gerätebetriebsweise in einen mandatorischen Steuerungszyklus eingeleitet wird, ohne daß der Patient dies als unangenehm wahrnimmt. Bei Erreichen einer vorgebbaren Schlaftiefe wird eine vollständige mandatorische Beatmung durchgeführt und hiermit eine möglichst weitgehende Verminderung der vom Patienten zu leistenden Atemarbeit erreicht.

Die Überleitung vom assistierten Beatmungszyklus zum mandatorischen Beatmungszyklus während der Einschlafphase erfolgt derart, daß keine Beeinträchtigung des Einschlafvorganges des Patienten erfolgt. Dies kann durch eine entsprechende Auswertung der Parameter zur Detektion des Schlafzustandes sichergestellt werden. In umgekehrter Weise erfolgt während eines Aufwachens des Patienten ein sanfter Übergang von der mandatorischen Beatmung zur assistierten Beatmung, so daß mit zunehmender Wachheit des Patienten der Anteil der mandatorischen Steuerung vermindert und der Anteil der assistierten Steuerung erhöht wird. Bei einer optimalen Abstimmung des Steuerungsablaufes auf die erfaßten Schlafparameter ist die Durchführung der mandatorischen Beatmung während des Schlafes im Bewußtsein des Patienten nicht wahrnehmbar.

Eine Überleitung des assistierten Beatmungszyklusses in einen mandatorischen Beatmungszyklus erfolgt derart, daß nach der Erkennung eines Schlafzustandes das Atem-Minuten-Volumen erhöht wird.

Eine möglichst starke Annäherung der Gerätebetriebsweise an einen natürlichen Beatmungsablauf des Patienten erfolgt dadurch, daß nach Erkennung des Schlafzustandes das Atem-Minuten-Volumen solange erhöht wird, bis es oberhalb der Spontanatmung liegt und kein spontaner Atemantrieb mehr gemessen wird.

Eine einfache Gerätestruktur wird dadurch bereitgestellt, daß eine Erkennung eines Schlafzustandes zeitgesteuert durchgeführt wird.

Eine kontinuierliche Veränderung der Gerätebetriebsweise wird dadurch unterstützt, daß eine Erkennung eines Schlafzustandes in Abhängigkeit von einer Messung einer Atmungsaktivität durchgeführt wird.

Darüber hinaus ist es auch möglich, daß eine Erkennung eines Schlafzustandes in Abhängigkeit von einer Messung einer EKG-Aktivität durchgeführt wird.

Ebenfalls ist daran gedacht, daß eine Erkennung eines Schlafzustandes in Abhängigkeit von einer Messung einer EEG-Aktivität, einer EMG-Aktivität, einer EOG-Aktivität oder von Aktigrafie-Daten durchgeführt wird.

Eine Bestimmung eines aktuellen Schlafzustandes kann dadurch qualitativ verbessert werden, daß eine Auswertung der Parameter zur Detektion des Schlafzustandes unter Verwendung eines neuronalen Netzes durchgeführt wird.

Zur Verbesserung einer Optimierung des Atem-Minuten-Volumens wird vorgeschlagen, daß eine Steuerung des Atem-Minuten-Volumens unter Verwendung einer Fuzzy-Logik durchgeführt wird.

Zur Unterstützung einer Erkennung des aktuellen Bedarfs wird vorgeschlagen, daß zyklisch das Atem-Minuten-Volumen abgesenkt wird, bis Spontanatmung gemessen wird.

Eine kontinuierliche Betriebsweise wird ebenfalls dadurch unterstützt, daß das Atem-Minuten-Volumen bei Erkennung einer Spontanatmung erhöht wird.

Ein vollautomatischer Steuerungsablauf mit Erkennung sowohl von Einschlafzuständen, Schlafzuständen sowie Aufwachphasen wird dadurch erreicht, daß zyklisch eine Auswertung auf Beibehaltung eines Schlafzustandes durchgeführt wird und daß bei Erkennung eines Verlassens des Schlafzustandes in den Grundzustand mit assistierten Beatmungszyklen zurückgekehrt wird.

In den Zeichnungen sind Ausführungsbeispiele der Erfindung schematisch dargestellt. Es zeigen:
- Fig. 1: Eine perspektivische Darstellung eines Beamtungsgerätes mit Verbindungsschlauch zu einer Beatmungsmaske und
- Fig. 2: ein schematisches Ablaufdiagramm zur Veranschaulichung der Verfahrensdurchführung sowie der Gerätesteuerung .

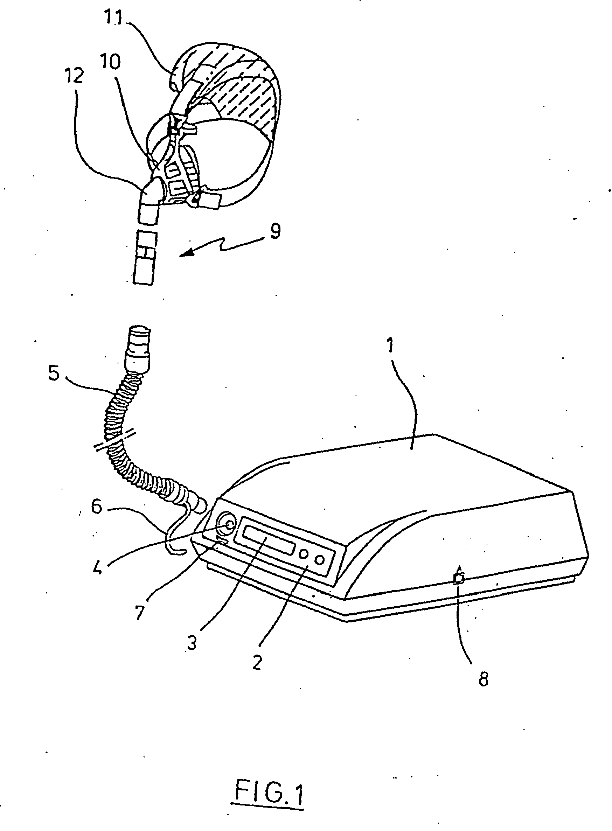

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

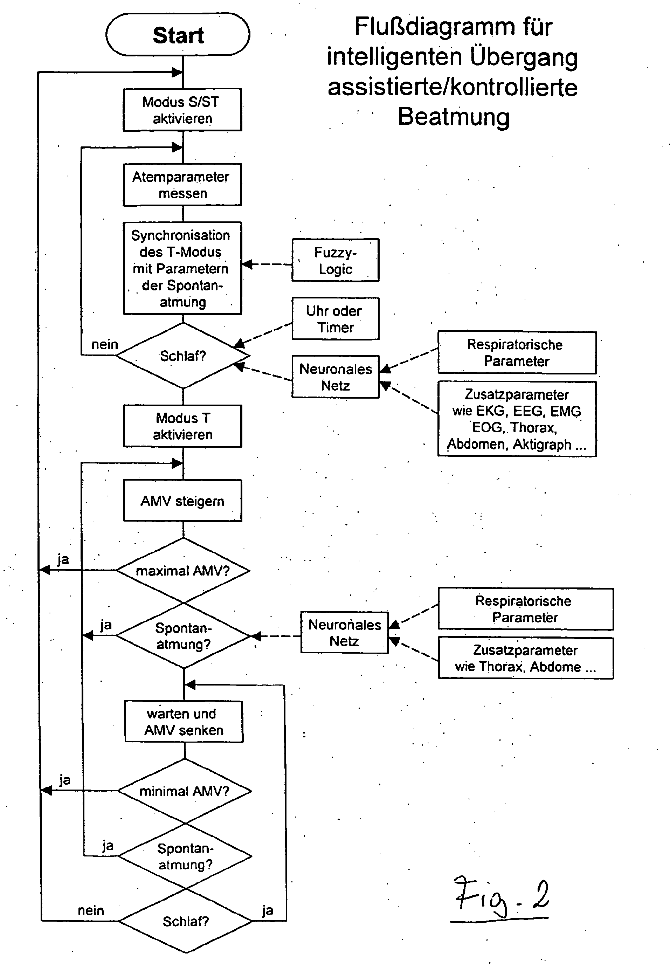

Fig. 2 veranschaulicht die Durchführung einer typischen Gerätesteuerung. Nach einem Einschalten des Gerätes wird zunächst in einem Grundzustand ein assistierter Beatmungszyklus unter Berücksichtigung mindestens eines meßtechnisch erfaßten Parameters zur Berücksichtigung einer eigenen Atmungsaktivität des Patienten durchgeführt. In Abhängigkeit von diesem erfaßten Atemparameter erfolgt eine Synchronisierung der Ansteuerung der Atemgasfördereinrichtung mit der Spontanatmung des Patienten. Die Auswertung des Atemparameters kann beispielsweise unter Verwendung einer Fuzzy-Logik erfolgen.

Ergibt eine weitere Parameterauswertung, daß kein Schlafzustand des Patienten vorliegt, so wird der Ablauf der Erfassung des Atemparameters sowie der Synchronisation der Funktion des Beatmungsgerätes und der Spontanatmung des Patienten zyklisch durchlaufen. Die Detektion eines Schlafzustandes kann beispielsweise zeitgesteuert oder über ein neuronales Netz zur Auswertung eines oder mehrerer spezieller Parameter erfolgen. Als Parameter zur Detektion eines Schlafzustandes kommen beispielsweise respiratorische Parameter und / oder Meßwerte zu EKG, EEG, EMG, EOG, Thoraxfunktion, Abdomen oder Aktigrafen in Frage.

Ergibt die Auswertung des Schlafzustandes, daß ein ausreichend tiefer Schlaf des Patienten vorliegt, so erfolgt ein Übergang von der assistierten Beatmung zu einer mandatorischen Beatmung. Es wird dann das Atem-Minuten-Volumen solange erhöht, bis das vom Beatmungsgerät vorgegebene Atem-Minuten-Volumen oberhalb des Spontan- Atem-Minuten-Volumens des Patienten liegt. Wird ein vorgegebenes maximales Atem-Minuten-Volumen erreicht, wird der erläuterte Ablauf erneut durchlaufen. Liegt das maximale Atem-Minuten-Volumen noch nicht vor, so erfolgt eine Analyse, ob eine Spontanatmung des Patienten vorliegt. Hierzu können über ein neuronales Netz respiratorische Parameter sowie Meßparameter im Hinblick auf Thoraxaktivitäten oder Abdome ausgewertet werden.

Wird eine Spontanatmung detektiert, erfolgt erneut eine Steigerung des Atem-Minuten-Volumens und eine Auswertung auf Erreichen des maximalen Atem-Minuten-Volumens. Liegt keine Spontanatmung vor, so wird das Atem-Minuten-Volumen abgesenkt und für einen vorgebbaren Zeitraum eine Warteschleife durchlaufen. Anschließend erfolgt eine Auswertung, ob ein minimales Atem-Minuten-Volumen erreicht ist. Liegt dies vor, so wird der erläuterte Vorgang wiederum von vorne durchlaufen. Liegt das minimale Atem-Minuten-Volumen nicht vor, erfolgt eine Auswertung, ob eine Spontanatmung erfolgt. Wird die Spontanatmung detektiert, so wird erneut das Atem-Minuten-Volumen gesteigert. Liegt keine Spontanatmung vor, erfolgt erneut die Auswertung auf Vorliegen eines Schlafzustandes. Liegt der Schlafzustand vor, wird wiederum das Atem-Minuten-Volumen gesenkt und die Warteschleife mit anschließender bereits erläuterter Auswertung durchlaufen. Liegt kein Schlafzustand vor, wird der gesamte vorstehende Ablauf erneut durchlaufen.

Durch die schrittweise Steigerung des Atem-Minuten-Volumens bzw. der Absenkung des Atem-Minuten-Volumens wird ein kontinuierlicher Übergang von einer assistierten Beatmung in eine mandatorische Beatmung bzw. von einer mandatorischen Beatmung in eine assistierte Beatmung erreicht.

Alternativ zur Darstellung in Fig. 1 kann auch ein Ausatemventil mit separatem Steuerschlauch oder einer entsprechenden Steuerleitung verwendet werden.

## Patentansprüche

1. Vorrichtung zur Beatmung, die mit einer Steuereinheit sowohl zur Vorgabe eines assistierten Beatmungszyklusses unter Berücksichtigung mindestens eines von einem Sensor erfaßten Meßsignals als auch zur Vorgabe eines zeitgesteuerten mandatorischen Beatmungszyklusses versehen ist und bei der die Steuereinheit ein Steuerungsprogramm aufweist, das in einem Grundzustand eine Atemgasfördereinrichtung in Abhängigkeit von den vom Sensor erfaßten Meßsignalen ansteuert und bei der die Steuereinheit mindestens einen Komparator aufweist, der bei Erkennung eines Schlafzustandes ein Steuersignal bereitstellt, gemäß dem die Steuereinheit die Atemgasfördereinrichtung mindestens teilweise entsprechend einem an die Parameter der Spontanatmung angepaßten mandatorischen Beatmungszyklus ansteuert, **dadurch gekennzeichnet, daß** das Steuerprogramm der Steuereinheit derart ausgebildet ist, daß die Steuereinheit in einem Wachzustand des Patienten den assistierten Beatmungszyklus aktiviert, eine zyklische Erfassung und Auswertung des Meßsignals zur Erkennung eines Schlafzustandes des Patienten durchgeführt, bei Erkennung eines Schlafzustandes des Patienten automatisch den mindestes teilweise mandatorischen Beatmungszyklus aktiviert und bei Erkennung einer zunehmenden Schlaftiefe des Patienten den Anteil des mandatorischen Beatmungszyklus durch Erhöhung eines Atem-Minuten-Volumens vorgibt.

2. vorrichtung nach Anspruch 1, **dadurch gekennzeichnet, daß** der Komparator an eine Zeitsteuerung angeschlossen ist.

3. Vorrichtung nach Anspruch 1 oder 2, **dadurch gekennzeichnet, daß** der Sensor zur Erfassung einer Atmungsaktivität ausgebildet ist.

4. Vorrichtung nach Anspruch 1 oder 2, **dadurch gekennzeichnet, daß** der Sensor zur Erfassung einer EKG-Aktivität ausgebildet ist.

5. Vorrichtung nach Anspruch 1 oder 2, **dadurch gekennzeichnet, daß** der Sensor zur Erfassung einer EEG-Aktivität ausgebildet ist.

6. Vorrichtung nach Anspruch 1 oder 2, **dadurch gekennzeichnet, daß** der Sensor zur Erfassung einer EMG-Aktivität ausgebildet ist.

7. Vorrichtung nach Anspruch 1 oder 2, **dadurch gekennzeichnet, daß** der Sensor zur Erfassung einer EOG-Aktivität ausgebildet ist.

8. Vorrichtung nach Anspruch 1 oder 2, **dadurch gekennzeichnet, daß** der Sensor zur Erfassung von aktigrafischen Daten ausgebildet ist.

9. Vorrichtung nach einem der Ansprüche 1 bis 8, **dadurch gekennzeichnet, daß** ein Synchronisator zur Anpassung der Ansteuerung der Atemgasfördereinrichtung an eine Spontanatmung eine Fuzzy-Logik aufweist.

10. Vorrichtung nach einem der Ansprüche 1 bis 9, **dadurch gekennzeichnet, daß** der Komparator ein neuronales Netz aufweist.

## Claims

1. Device for breathing, which is connected to a control unit both for pre-setting an assisted breathing cycle taking account of at least one measurement signal detected by a sensor and for pre-setting a time-controlled mandatory breathing cycle, and the control unit has a control programme which activates a device for delivering breathing gas in response to measurement signals detected by the sensor in a basic state, and the control unit has at least one comparator which issues a control signal on detecting a sleeping state, on the basis of which the control unit activates the device for delivering breathing gas at least partly corresponding to a mandatory breathing cycle adapted to the parameter for spontaneous breathing, **characterised in that** the control programme of the control unit is configured so that the control unit activates the assisted breathing cycle when the patient is in an awake state, runs a cyclic detection and evaluation of the measurement signal in order to detect when the patient is in a sleeping state, on detecting that the patient is in a sleeping state automatically activates the at least partially mandatory breathing cycle, and on detecting that the patient is sleeping more deeply pre-sets the proportion of the mandatory breathing cycle by increasing a volume of breath per minute.

2. Device as claimed in claim 1, **characterised in that** the comparator is connected to a time control system.

3. Device as claimed in claim 1 or 2, **characterised in that** the sensor is configured to detect a breathing activity.

4. Device as claimed in claim 1 or 2, **characterised in that** the sensor is configured to detect an ECG activity.

5. Device as claimed in claim 1 or 2, **characterised in that** the sensor is configured to detect an EEG activity.

6. Device as claimed in claim 1 or 2, **characterised in that** the sensor is configured to detect an EMG activity.

7. Device as claimed in claim 1 or 2, **characterised in that** the sensor is configured to detect an EOG activity.

8. Device as claimed in claim 1 or 2, **characterised in that** the sensor is configured to detect actigraphic data.

9. Device as claimed in one of claims 1 to 8, **characterised in that** a synchroniser has a fuzzy logic for adapting activation of the device for delivering breathing gas to a spontaneous breathing.

10. Device as claimed in one of claims 1 to 9, **characterised in that** the comparator has a neural network.

## Revendications

1. Appareil respiratoire qui est équipé d'une unité de commande aussi bien pour la prédétermination d'un cycle de respiration assisté, compte tenu d'au moins un signal de mesure saisi par un capteur, que pour la prédétermination d'un cycle de ventilation forcée à commande temporelle, et dans lequel l'unité de commande présente un programme de commande qui, dans un état de base, actionne un dispositif d'amenée de gaz respiratoire en fonction des signaux de mesure saisis par le capteur, et dans lequel l'unité de commande présente au moins un comparateur qui, lors de la détection d'un état de sommeil génère un signal de commande, conformément auquel l'unité de commande actionne le dispositif d'amenée de gaz respiratoire au moins partiellement, conformément à un cycle de ventilation forcée, adapté aux paramètres de la respiration spontanée, **caractérisé en ce que** le programme de commande de l'unité de commande est conçu de telle manière que l'unité de commande active le cycle de ventilation assistée, lorsque le patient est à l'état éveillé, effectue un captage et une évaluation cycliques du signal de mesure en vue de la détection d'un état de sommeil du patient, active automatiquement le cycle de ventilation forcée, au moins partiellement, lors de la détection d'un état de sommeil du patient, et, lors de la détection d'une profondeur croissante du sommeil du patient, prédétermine la part du cycle de respiration forcée par augmentation d'un volume de respiration/minute.

2. Dispositif selon la revendication 1, **caractérisé en ce que** le comparateur est raccordé à une commande temporelle.

3. Dispositif selon revendication 1 ou 2, **caractérisé en ce que** le capteur est conçu pour la détection d'une activité respiratoire.

4. Dispositif selon revendication 1 ou 2, **caractérisé en ce que** le capteur est conçu pour la détection d'une activité ECG.

5. Dispositif selon revendication 1 ou 2, **caractérisé en ce que** le capteur est conçu pour la détection d'une activité EEG.

6. Dispositif selon revendication 1 ou 2, **caractérisé en ce que** le capteur est conçu pour la détection d'une activité EMG.

7. Dispositif selon revendication 1 ou 2, **caractérisé en ce que** le capteur est conçu pour la détection d'une activité EOG.

8. Dispositif selon revendication 1 ou 2, **caractérisé en ce que** le capteur est conçu pour la saisie de données d'actigrammes.

9. Dispositif selon l'une des revendications 1 à 8, **caractérisé en ce que**, pour l'adaptation de l'actionnement du dispositif de transport de gaz respiratoire à une respiration spontanée, un synchronisateur présente une logique floue.

10. Dispositif selon l'une des revendications 1 à 9, **caractérisé en ce que** le comparateur présente un réseau neuronal.
